# EUROPEAN PATENT SPECIFICATION

(11) **EP 0 542 860 B1**
(45) Date of publication and mention of the grant of the patent: **20.05.1998**
(21) Application number: 91915005.2
(22) Date of filing: 07.08.1991
(51) Int. Cl.: A61K 31/40, B65G 57/10

(54) **IMMUNOSUPPRESSIVE COMPOSITIONS**
IMMUNOSUPPRESSIVE ZUSAMMENSETZUNGEN
COMPOSITIONS IMMUNOSUPPRESSIVES

(30) Priority: 10.08.1990 US 565826; 05.12.1990 US 622452
(43) Date of publication of application: 26.05.1993
(73) Proprietor: AnorMED Inc, Langley BC V2Y 1N5 (CA)
(72) Inventor: BADGER, Alison Mary, Bryn Mawr, PA 19010 (US)
(74) Representative: Wishart, Ian Carmichael
(86) International application number: US9105619
(87) International publication number: WO9202229

(56) References cited:
- US-A- 4 963 557
- Journal of Pharmacology and Experimental et al., Therapeutics, Vol. 2, No. 1, 03 October 1985; D. MARTINO et al.: "Antiarthritic and Immuniegulatory Activity of spirogermanium", pages 103-110
- Immuniohaiemacology, Vol. 10, 1985; BADGN et al.: "Generation of suppresser cells in novel hetenscyclic anticancer drug", pp. 201-200
- CHEMICAL ABSTRACTS, Vol. 110, No. 69098V, issued 27 February 1989; YOSHIMURA et al.: "Effect of the new immunisuppressive agent. Fr 506 on lymphokive production and responsiveness of human peripheral blood lymphocytes", see page 26, column 4, Abstract 690980 Igaku no Ayumi, 1983, Vol. 147 (a), 783-4 (Japan)
- CHEMICAL ABSTRACTS, Vol. 87, No. 15, issued 10 October 1977; MARTEL et al.: "Inhibition of the Immune response by Rapamycin, a new antifungal antibiotic", see page 90, column 1, Abstract 111825p, Can. J. Physiol Pharmacol, 1977, Vol. 55 (1), Vol. 55 (1), 48-51
- CHEMICAL ABSTRACTS, Vol. 95, No. 21, issued 23 November 1981; BUNJES et al.: "Cyclosporiua mediates mediates immuno suppression of primary cyctotoxic T cell response by impairing the release of Interleukin 1 and Interlive 2", see page 28, column 1, Abstract 180734K, Eur J Immunol 1981, Vol. 11 (8) pages 657-61

## Description

This invention relates to a pharmaceutical composition containing a non-specific (NS) suppressor cell inducing compound and a non-NS suppressor cell inducing immunosuppressive compound and a pharmaceutically acceptable carrier or diluent.

### BACKGROUND OF THE INVENTION

Conventional drugs used in the treatment of autoimmune diseases and graft/transplantation rejection, such as cyclosporin A, corticosteroids, azathioprine, polyclonal anti-lymphocyte globulins and monoclonal T cell antibodies are somewhat effective in electing an immunosuppressive response. However, their toxicity profiles frequently limit their clinical benefit. Thus, the treatment of autoimmune diseases, graft/transplantation rejection and other maladies requiring immunosuppression with agents having low-toxicity profiles remains a major clinical problem.

The use of monoclonal anti-IL2 receptor antibodies in combination with cyclosporin A has been reported. See, Diamantstein, T, et al., (1986) Immunobiol. 172:391-399; Kupiec-Weglinski, J.W., et al., (1988) Transplant Proc 20:207-216 and Hancock, W.W., et al., (1990), Transplantation 49:416-421. The use of bromocriptine in combination with cyclosporin A (Carrier, M., et al., (1990), Ann. Thorac. Surg. 49:129-32)and thalidomide in combination with cyclosporin A (Tamura, F., et al., (1990) transplantation 49:20-25) has also been reported.

Non-specific suppressor cell inducing compounds are agents which induce the production of a population of natural suppressor cells which do not have the characteristics of mature T cells, B cells, macrophages or natural killer cells and are therefore of the null or non-specific phenotype. Natural suppressor cells are capable of inhibiting a variety of immune responses in vivo.

The immunosuppressive activity associated with total lymphoid irradiation (TLI) has been attributed to the generation of a population(s) of natural suppressor cells. See, Strober, S., (1984) Ann. Rev. Immun. 2:219 and Maier, T., et al., (1986), Immunol. Today 7:312. The use of TLI as part of a combination treatment with cyclosporine and either cyclophosphamide (See; Yamaguchi, Y., et al., (1990), Transplantation 49:13-17) or splenectomy (See, Miyamura, K., et al., (1988), Bone Barrow Transplantation 3:457-461) has been reported.

Classes of compounds known to induce NS suppressor cells include N-amino alkyl azaspirogermanium alkanes (See, e.g., Badger, A., et al., (1985), Immunopharm. 10:201 and DiMartino, M.J., et al., (1986), J. Pharm. Exp. Ther. 236:103) and N-amino alkyl azaspiro alkanes (See, e.g., Badger, A., et al., (1989), Int. J. Immunopharmac. 11:839-846 and European Patent Application Publication Number 0310321 A2).

It has now been discovered that combining a NS suppressor cell inducing compound with a non-NS suppressor cell inducing immunosuppressive compound increases immunosuppressive activity in vivo to an extent beyond which either compound achieves alone or would be expected to achieve when combined.

### SUMMARY OF THE INVENTION

This invention relates to a pharmaceutical composition containing a NS suppressor cell inducing compound and a non-NS suppressor cell inducing immunosuppressive compound such as cyclosporin A.

### DETAILED DESCRIPTION OF THE INVENTION

By the term "cyclosporin A" as used herein is meant the cyclic polypeptide of the formula 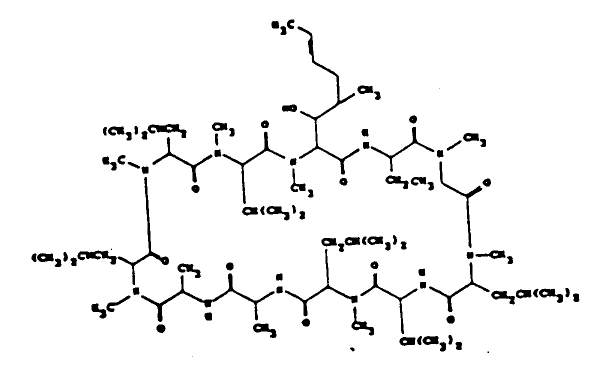 Cyclosporin A is produced as a metabolite by the fungus species Tolypocladium inflatum Gams. Chemically, cyclosporin A is designated as
[R-[R,R-(E)]]-cyclic-(L-alanyl-D-alanyl-N-methyl-L-leucyl-N-methyl-L-leucyl-N-methyl-L-valyl-3-hydroxyN, 4-dimethyl-L-2-amino-6-octenoyl-L-α-amino-butyryl-N-methylglycyl-N-methyl-L-leucyl-L-valyl-N-methyl-L-leucyl).

Cyclosporin A is commercially available in oral dosage form or intravenous dosage form under the trade name Sandimmune^{Θ} and is manufactured by Sandoz Ltd., Basle Switzerland for Sandoz Pharmaceuticals Corporation, East Hanover, N.J. 07936. The total synthesis of cyclosporin A has been reported by Wenger, Transplant Proc. 15 (4), suppl. 1, 2230 (1983).

By the term "FK-506" as used herein is meant the macrolide immunosuppressant of the formula: 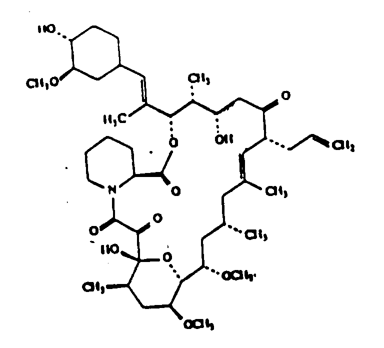 FK-506 is isolated from Streptomyces tsukubaensis No. 9993 and is claimed in European Patent Application No. 184,162 (1986 to Fujisawa). Chemically, FK-506 is designated as [3S-[3R*[E(1S*,3S*,4S*)],4S*,5R*,8S*,9E,12R*,14R*,15S*,16R*, 18S*^{,}19S*,26aR*]]-5,6,8,11,12,13,14,15,16,17,18,19,24,25,26, 26a-Hexadecahydro-5,19-dihydroxy-3-[2-(4-hydroxy-3-methoxycyclohexyl)-1-methylethenyl]-14,16-dimethoxy-4,10,-12,18-tetramethyl-8-(2-propenyl)-15,19-epoxy-3H-pyrido[2,1-c][1,4]oxaazacyclotricosine-1,7,20,21(4H,23H)-tetrone.

By the term "SPERGUALIN" as used herein is meant the compound of the formula: 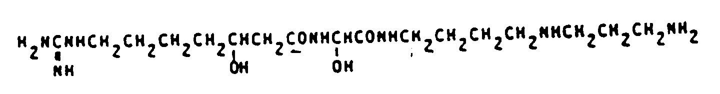 Spergualin is isolated from Bacillus Laterosporus and is a known immunosuppressive agent. (see, Ameniya, H et al., (1990), Transplantation 49:337:343).

By the term "RAPAMYCIN" as used herein is meant the macrolide immunosuppressant of the formula: 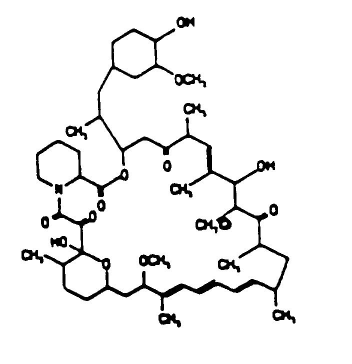 Rapamycin is a fermentation product of Streptomyces Hygroscopicus and was first reported in 1975 (J. Antibiot 28:727 (1975).

By the term "RS-61443" as used herein is meant a prodrug of mycophenolic acid. RS-61443 is a known immunosuppressive agent under investigation by Syntex.

By the term "non-NS suppressor cell inducing immunosuppressive compound" as used herein is meant any immunosuppressive agent that does not induce the in vivo production of a population of natural or non-specific suppressor cells.

Preferred non-NS suppressor cell inducing compounds for use in the compositions and methods of the invention include: FK-506, spergualin, rapamycin, RS-61443 and cyclosporin A.

By the term "NS suppressor cell inducing compound" as used herein is meant any compound which induces the in vivo production of a population of natural suppressor cells. Such natural suppressor cells do not have the characteristics of mature T cells, B cells, macrophages or natural killer cells and are therefore of the null or non-specific phenotype. Natural suppressor cells are capable of inhibiting a variety of immune responses in vivo.

Preferred NS suppressor cell inducing compounds for use in the compositions and methods of the invention include the N-aminoalkyl azaspirogermanium alkanes reported by Badger et al., Immunopharm., 10:201 (1985) and DiMartino et al., J. Pharm. Exp, Ther., 236:103 (1986), including:
N-(3-dimethylaminopropy])-2-aza-8,8-dimethyl-8-germanspiro[4,5]decane;
N-(3-dimethylaminopropyl)-2-aza-8,8-diethyl-8-germanspiro[4,5]decane;
N-(3-dimethylaminopropyl)-2-aza-8,8-dipropyl-8-germanspiro[4,5]decane; and
N-(3-dimethylaminopropy])-2-aza-8,8-dibutyl-8-germanspiro[4,5]decane.

Additional preferred NS suppressor cell inducing compounds for use in the composition and method of the invention include the N-aminoalkylazaspiro alkanes described in U.S. Patent 4,963,557 filed September 13, 1988 for example, compounds of the formula: 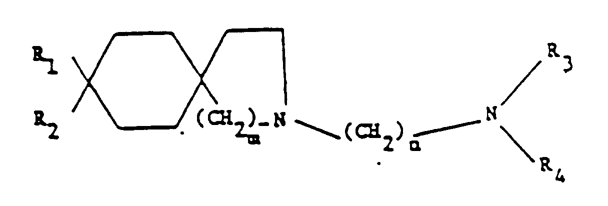 wherein:
n is 3-7;
m is 1 or 2;
R₁ and R₂ are the same or different and are selected from hydrogen or straight chain, branched chain or cyclic alkyl, provided that the total number of carbon atoms contained by R₁ and R₂ when taken together is 4-10; or R₁ and R₂ are joined together to form a cyclic alkyl group containing 3-7 carbon atoms;
R₃ and R₄ are the same or different and are selected from hydrogen or straight chain alkyl containing 1-3 carbon atoms; or R₃ and R₄ are joined together to form a cyclic alkyl group containing 4-7 carbon atoms;
provided that when R₁ and R₂ are CH₃CH₂, R₃ and R₄ are CH₃ and m is 1 or 2, n is other than 3; and further provided that when R₁ is H; R₂ is (CH₃)₃C; R₃ and R₄ are CH₃ and m is 1 or 2, n is other than 3; and further provided that when R₂ is H; R₁ is (CH₃)₃C; R₃ and R₄ are CH₃ and m is 2, n is other than 3;
or a pharmaceutically acceptable salt, hydrate or solvate thereof.
Especially preferred are the following N-aminoalkyl azaspiro alkane derivatives: 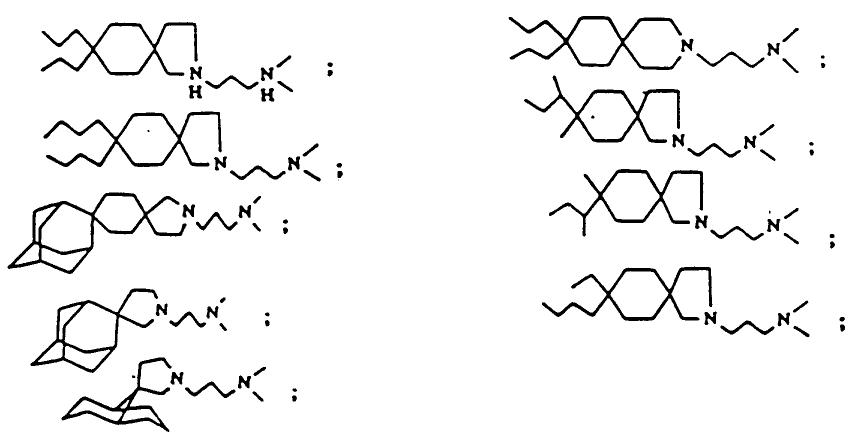 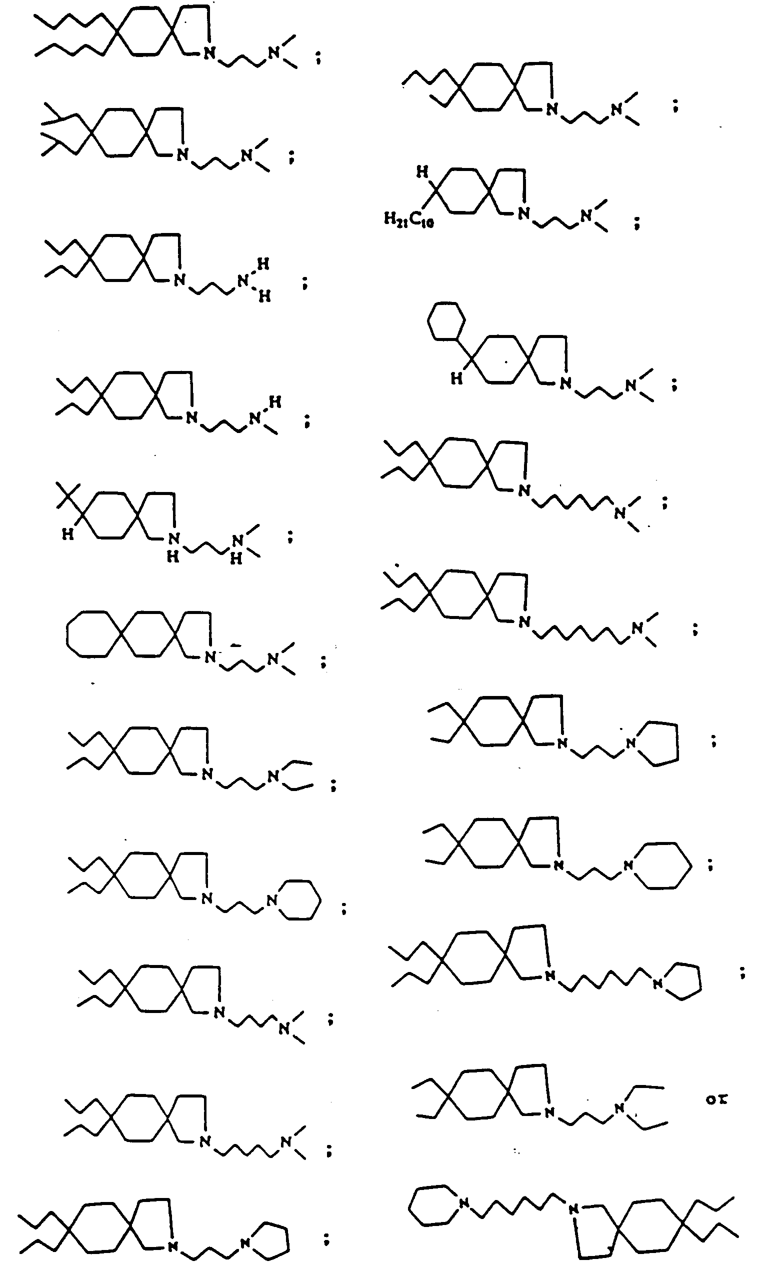 or a salt thereof.

The most preferred NS suppressor cell inducing compound is N,N-dimethyl-8,8-dipropyl-2-azaspiro[4,5]-decane-2-propanamine, (hereinafter referred to as 'Compound A')

Persons skilled in the art can readily determine if a compound other than ones specifically referred to herein induces non-specific suppressor cell activity by utilizing the assay described in Badger et al., Immunopharm, 16, 33-43 (1988), Thus, all such compounds are therefore included within the scope of the term 'non-specific suppressor cell inducing compound' as used herein.

By the term "administering an effective amount of a NS suppressor cell inducing compound and a non-NS suppressor cell inducing immunosuppressive compound" as used herein is meant either simultaneous administration or any manner of consecutive administration, i.e., either the non-NS suppressor cell inducing immunosuppressive compound or the non-specific suppressor cell inducing compound may be administered first. Preferably, if the administration is not simultaneous, the two compounds are administered in a close time proximity to each other. Furthermore, it does not matter if the compounds are both administered in the same dosage form, e.g., the non-NS suppressor cell cell inducing immunosuppressive compound may be administered by injection and the non-specific suppressor cell inducing compound may be administered orally.

The therapeutic efficacy of a pharmaceutical composition of the invention was tested according to the procedure of Kupiec-Weglinski, J.W., et al., (1988) Transplant Proc. 20:207-216 (hereinafter "Kupiec-Weglinski") to determine its in vivo potency as an acute transplantation/graft rejection modulator.

Briefly, to preform these experiments, the following protocol was employed. Male inbred adult rats (Lewis-Brown Norway F1 Hybrid rats, obtained from Harlan sprague - Dawley Inc., Indianapolis, Indiana) were used. Lewis-Brown Norway F1 Hybrid cardiac allografts were transplanted to the abdominal great vessels of Lewis recipients. The animals were administered compounds according to the following five distinct regimens.
- Protocol I.: Pretreatment Protocol - 20 mg/kg/day of Compound A was administered orally for 7 consecutive days prior to transplantation (days -7 to -1).
- Protocol II: Treatment Protocol - 20 mg/kg/day of Compound A was administered orally for 7 consecutive post-transplant days (days 0-6) followed by intermittent 5 day courses (days 9-13 and 16-20).
- Protocol III: Pretreatment and Treatment Protocol - 20 mg/kg/day of Compound A was administered orally for 7 consecutive days prior to transplantation (days -7 to -1) followed by intermittent 5 day courses (days 9-13 and 16-20).
- Protocol IV: Combination Protocol - 20 mg/kg/day of Compound A was administered orally and 1.5 mg/kg/day of cyclosporin A was administered by injection for 7 consecutive post-transplant days (days 0-6).
- Protocol V: Untreated.

Ventricular contractions were assessed daily by palpation through the recipient flank. Rejection was defined as the day of cessation of myocardial contractions and confirmed histologically.

In the rats treated with a single compound (i.e., pretreatment protocol, treatment protocol or pretreatment and treatment protocol), the graft survival time was increased from an average of 7 days in the untreated model to an average of 16 days using Compound A. Cyclosporin A, at a dose of 1.5 mg/kg/day, when used alone in this model increased the graft survival time from an average of 7 days in the untreated model to an average of 12 days. (See, Kupiec-Weglinski). Alternatively, in the rats treated with the combination protocol, the transplants were routinely retained for over 40 days. To increase the graft survival time to equal that of the Combination Protocol using cyclosporin A alone requires a dosage an order of magnitude higher than that used in the combination protocol, i.e., 15 mg/kg/day (See, Kupiec-Weglinski). Thus, the administration of the claimed pharmaceutical composition was synergistic, i.e., it extended the graft survival time significantly longer than can be obtained by either compound. The efficacious dosage of cyclosporin A was reduced by one order of magnitude. Since cyclosporin A is known to be highly nephrotoxic, and the level of nephrotoxicity increases with increasing dosages, reducing the dosage cyclosporin A required to obtain the desired immunosuppressive effect is highly desirable. Thus, it is expected that the discovery that the compositions and method of this invention are synergistic will enable the treatment of disorders requiring immunosuppression with the efficacy of conventional treatment methods but with lower toxicity problems. The results of the above experiment are summarized in the following Table I.

**TABLE I**

| Effects of Various Treatment Protocols with Compound A on Lewis Rat Recipients of Lewis-Brown Norway F1 hybrid Cardiac Allografts. | | | |
|---|---|---|---|
| | N | GRAFT SURVIVAL (days) | MEAN±SD |
| Protocol I | 7 | 10,11,12,13,14,16,27 | 14.7 ± 5.8 |
| Protocol II | 7 | 14,16,16,16*,16*,19,20 | 16.7 ± 2.0 |
| Protocol III | 7 | 8,15,16,16,16,19,22 | 16.0 ± 4.3 |
| Protocol IV | 3 | 42,42,indefinite survival | |
| Protocol V (Untreated) | 7 | 6,7,7,7,8,8,8 | 7.3 ± 0.8 |
| N is the number of rats tested | | | |

| | | | |
|---|---|---|---|
| * graft survival in hosts treated for 7 post-transplant days only | | | |

The data in the above Table 1 clearly demonstrates the synergistic effects of a combination of cyclosporin A and a non-specific suppressor cell inducing compound on the rejection of cardiac allografts in adult male rats.

The claimed pharmaceutical compositions are incorporated into convenient dosage forms such as capsules, tablets, or injectable preparations. Solid or liquid pharmaceutical carriers are employed. Solid carriers include starch, lactose, calcium sulfate dihydrate, terra alba, sucrose, talc, gelatin, agar, pectin, acacia, magnesium stearate, and stearic acid. Liquid carriers include syrup, peanut oil, olive oil, saline, and water. Similarly, the carrier or diluent may include any prolonged release material, such as glyceryl monostearate or glyceryl distearate, alone or with a wax. The amount of solid carrier varies widely but, preferably, will be from about 25 mg to about 1 g per dosage unit. When a liquid carrier is used, the preparation will be in the form of a syrup, elixir, emulsion, soft gelatin capsule, sterile injectable liquid such as an ampoule, or an aqueous or nonaqueous liquid suspension.

The pharmaceutical preparations are made following conventional techniques of a pharmaceutical chemist involving mixing, granulating, and compressing, when necessary, for tablet forms; or mixing, filling and dissolving the ingredients, as appropriate, to give the desired oral or parenteral products.

The pharmacokinetic properties of each active component of the pharmaceutical composition of the invention must be contemplated when formulating conventional dosage regimens. Both components can be incorporated into a timed release dosage unit form in which several doses are treated for delayed or sustained release of the medicament. Such dosage units may comprise sustained release granules, sugar centered spheres or multilayered tablets in each of which the availability of the active ingredient is controlled by coating with a lupid or polymeric material.

The claimed pharmaceutical composition and method are useful in effecting immunosuppression in mammals. Thus, they have therapeutic and/or prophylactic utility in treating diseases and conditions wherein inducing an immune suppression response is desired. Such diseases and conditions include, but are not limited to, Rheumatiod arthritis, systemic lupus erythematosis, multiple sclerosis, acute transplantation/graft rejection, myasthenia gravis, progressive systemic sclerosis, multiple myeloma, atopic dermatitis, hyperimmunoglobin E, hepatitis B antigen negative chronic active hepatitis, Hashimoto's thyroiditis, Familial Mediterranean fever, Grave's disease, autoimmune hemolytic anemia, primary biliary cirrhosis, insulin dependent diabetes mellitus and inflammatory bowel disease.

To maximize its synergistic effect, the individual compounds of the claimed combinations can be administered as a single pharmaceutical composition or consecutively in separate pharmaceutical compositions. One of skill in the art using conventional techniques can determine the most appropriate way to administer the two compounds (consecutively versus simultaneously) depending on such factors as the age, sex, weight and health of the patient and the disease state to be treated. Also, during therapy the appropriate administration scheme will be apparent to one of skill in the art.

The dose of the NS suppressor cell inducing compounds to be used in the method of the invention is selected from the range of 0.01 - 1000 mg/kg of body weight per day, preferably 0.01-100 mg/kg. The dose of the non-NS suppressor cell inducing immunosuppressive compound to be used in the method of the invention is selected from the range of 0.01 mg/kg - 20 mg/kg of body weight per day, preferably 0.01 mg/kg - 2.0 mg/kg.

The selected dose is administered to a mammal in need of immounosuppression from 1-6 times daily, and is administered topically, orally, rectally, by injection, or continuously by infusion. The administration route which is most appropriate is readily determined by one of skill in the art using conventional techniques. Oral dosage units for human administration preferably contain from 0.01 to 500 mg of each active compound Oral administration, which uses lower dosages is preferred. Parenteral administration, at higher dosages, however, also can be used when safe and convenient for the patient.

The following examples illustrate preparation of the claimed pharmaceutical compositions. The examples are not intended to limit the scope of the invention as defined hereinabove and as claimed below.

### EXAMPLE 1 - Hard Gelatin Capsule

An oral dosage form for administering the claimed compounds and compositions is produced by screening, mixing and filling into hard gelatin capsules the ingredients in the proportions shown in Table II below.

**Table II**

| Ingredients | Amounts |
|---|---|
| N,N-dimethyl-8,8-dipropyl-2-azaspiro[4,5]decane-2-propanamine | 10 mg |
| Cyclosporin A | 100 mg |
| Magnesium stearate | 2 mg |
| Lactose | 30 mg |
| Starch | 10 mg |

### EXAMPLE 2 - Tablet

The microcrystalline cellulose, lactose and claimed compounds listed in Table III below, are screened, mixed and granulated in the proportions shown with a 10% gelatin solution. The wet granules are screened, dried, mixed with the starch, talc and stearic acid, screened and compressed into a tablet.

**Table III**

| Ingredients | Amounts |
|---|---|
| N,N-dimethyl-8,8-dipropyl-2-azaspiro[4,5]decane-2-propanamine | 10 mg |
| Cyclosporine A | 100 mg |
| Microcrystalline cellulose | 30 mg |
| Lactose | 30 mg |
| Starch | 10 mg |
| Talc | 4 mg |
| Stearic acid | 4 mg |

### EXAMPLE 3 - Injectable Preparation

Cyclospirin A and N,N-dimethyl-8,8-dipropyl-2-azaspiro[4,5]decane-2-propanamine are solubilized or dispersed in oil based systems shown below to prepare an injectable formulation:

| Ingredients | Amounts |
|---|---|
| N,N-dimethyl-8,8-dipropyl-2-azaspiro[4,5]decane-2-propanamine | 10 mg |
| Cyclosporine A | 100 mg |
| Polyoxyethylated Castor Oil or Arachis Oil | 13 mg |
| Alcohol | 33% v/v |
| Sterile Water for Injection | 1 ml |

### EXAMPLE 4

The following compounds (expressed as base weight) are mixed together with 30 mg of lactose, 2 mg of magnesium stearate and 10 mg starch then filled into a hard gelatin capsule. These capsules are administered from 1-6 times daily to a patient in need of immunosuppressive activity.
A. N,N-dimethyl-8,8-dipropyl-2-azaspiro[4,5]decane-2-propanamine 10 mg; cyclosporin A 100 mg.
B. N,N-dimethyl-8,8-diethyl-2-azaspiro[4,5]decane-2-propanamine 10 mg; cyclosporin A 100 mg.
C. N-(3-dimethylaminopropy])-2-aza-8-diethyl-8-germanspiro[4,5]decane 10 mg; cyclosporin A 100 mg.

### EXAMPLE 5 - Soft Gelatin Capsule

An oral dosage form for administering the claimed compounds and compositions is produced by dispersing claimed compounds in polyethylene glycol vegetable oil and filling into soft gelatin capsules:

| Ingredients | Amounts |
|---|---|
| N,N-dimethyl-8,8-dipropyl-2-azaspiro[4,5]decane-2-propanamine | 10 mg |
| Cyclosporin A | 100 mg |
| Polyehtylene Glycol | 50 mg |
| Vegetable Oil | 50 mg |

### EXAMPLE 6 - Micorparticulate System

The microcyrystalline cellulose, starch, and claimed compounds listed below are screened, mixed and spheronized in the proportions shown with a 10% gelatin solution. The wet particles are screened, dried, and sized. The resulting dried particles can be film-coated and filled into a hard gelatin capsule or compressed into a tablet with the addition of magnesium stearate.

| Ingredients | Amounts |
|---|---|
| N,N-dimethyl-8,8-dipropyl-2-azaspiro[4,5]decane-2-propanamine | 10 mg |
| Cyclosporin A | 100 mg |
| Microcrystalline Cellulose | 50 mg |
| Starch | 10 mg |
| Magnesium Stearate | 2 mg |

### EXAMPLE 7 - Oral Solution

An oral solution dosage form for administering the claimed compounds and compositions is produced by disolving claimed compounds in propylene glycol and mixing with remaining ingredients in the following proportions shown below:

| Ingredients | Amounts |
|---|---|
| N,N-dimethyl-8,8-dipropyl-2-azaspiro[4,5]decane-2-propanamine | 10 mg |
| Cyclosporin A | 100 mg |
| Sorbitol Solution | 10 mg |
| Propylene Glycol | 40 mg |
| Purified Water | 40 mg |

### EXAMPLE 8 - Oral Emulsion

An oral emulsion dosage form for administering the claimed compounds and compositions is produced by dissolving claimed compunds in vegetable oil. The polyoxyethylene sorbitan ester and sorbitol solution are dissolved in water, and the oil mixture is dispersed in the water mixture forming an oil-in-water emulsion.

| Ingredients | Amounts |
|---|---|
| N,N-dimethyl-8,8-dipropyl-2-azaspiro[4,5]decane-2-propanamine | 10 mg |
| Cyclosporin A | 100 mg |
| Vegetable Oil | 50 mg |
| Polyoxyethylene Sorbitan Ester | 20 mg |
| Sorbitol Solution | 10 mg |
| Purified Water | 100 mg |

While the preferred embodiments of the invention are illustrated by the above, it is to be understood that the invention is not limited to the precise instructions herein disclosed and that the right to all modifications coming within the scope of the following claims is reserved.

## Claims (Claims for the following Contracting State(s): AT, BE, CH, DE, DK, FR, GB, IT, LI, LU, NL, SE)

1. A composition comprising:
(a) a non-specific (NS) suppressor cell inducing compound, and
(b) a non-NS suppressor cell inducing immunosuppressive compound.

2. The composition of Claim 1 in which the quantity of the non-specific suppressor cell inducing compound is selected from 0.01-1000 mg/kg and the quantity of the non-NS suppressor cell inducing immunosuppressive compound is selected from 0.01 - 20 mg/kg.

3. The composition of Claim 1 in which the non-specific suppressor cell inducing compound is N,N-diethyl-8,8-dipropyl-2-azaspiro[4,5]-decane-2-propanamine or N,N-dimethyl-8,8-dipropyl-2-azaspiro [4,5]-decane-2-propanamine and the non-NS suppressor cell inducing immunosuppressive compound is: FK-506, spergualin, rapamycin, RS-61443 or cyclosporin A.

4. The composition of Claim 1 in which the non-specific suppressor cell inducing compound is N,N-diethyl-8,8-dipropyl-2-azaspiro[4,5]-decane-2-propanamine and the non-NS suppressor cell inducing immunosuppressive compound is cyclosporin A.

5. A pharmaceutical composition comprising a composition according to anyone of Claims 1 to 4 and a pharmaceutically acceptable carrier.

6. A composition according to any one of Claims 1 to 5 for use as an active therapeutic substance.

7. A composition according to any one of Claims 1 to 5 for use in inducing immunosuppressive activity.

8. Use of a composition according to any one of Claims 1 to 5 in the manufacture of a medicament for use in inducing immunosuppressive activity.

9. Use of a non-specific (NS) suppressor cell inducing compound in the manufacture of a medicament for inducing immunosuppression with the consecutive or simultaneous administration of a non-NS suppressor cell inducing immunosuppressive compound.

10. Use of a non-NS suppressor cell inducing immunosuppressive compound in the manufacture of a medicament for inducing immunosuppression with the consecutive or simultaneous administration of a non-specific (NS) suppressor cell inducing compound.

11. A use according to claim 9 or 10 wherein the quantity of the non-specific suppressor cell inducing compound is selected from 0.1-1000 mg/kg and the quantity of the non-NS suppressor cell inducing immunosuppresssive compound is selected from 0.01-20 nmg/kg.

12. A use according to claim 9 or 10 wherein the non-specific suppressor cell inducing compound is N,N-diethyl-8,8-dipropyl-2-azaspiro[4,5]-decane-2-propanamine or N,N-dimethyl-8,8-dipropyl-2-azaspiro[4,5]-decane-2-propanamine and the non-NS suppressor cell inducing immunosuppressive compound is: FK-506, spergualin, rapamycin, RS-61443 or cyclosporin A.

13. A use according to claim 12 wherein the non-specific suppressor cell inducing compound is N,N-diethyl-8,8-dipropyl-2-azaspiro[4,5]-decane-2-propanamine and the non-NS suppressor cell inducing immunosuppressive compound is cyclosporin A.

## Claims (Claims for the following Contracting State(s): ES, GR)

1. Use of a non-specific (NS) suppressor cell inducing compound in the manufacture of a medicament for inducing immunosuppression with the consecutive or simultaneous administration of a non-NS suppressor cell inducing immunosuppressive compound.

2. Use of a non-NS suppressor cell inducing immunosuppressive compound in the manufacture of a medicament for inducing immunosuppression with the consecutive or simultaneous administration of a non-specific (NS) suppressor cell inducing compound.

3. A use according to claim 1 or 2 wherein the quantity of the non-specific suppressor cell inducing compound is selected from 0.1-1000 mg/kg and the quantity of the non-NS suppressor cell inducing immunosuppresssive compound is selected from 0.01-20 nmg/kg.

4. A use according to claim 1 or 2 wherein the non-specific suppressor cell inducing compound is N,N-diethyl-8,8-dipropyl-2-azaspiro[4,5]-decane-2-propanamine or N,N-dimethyl-8,8-dipropyl-2-azaspiro[4,5]-decane-2-propanamine and the non-NS suppressor cell inducing immunosuppressive compound is: FK-506, spergualin, rapamycin, RS-61443 or cyclosporin A.

5. A use according to claim 4 wherein the non-specific suppressor cell inducing compound is N,N-diethyl-8,8-dipropyl-2-azaspiro[4,5]-decane-2-propanamine and the non-NS suppressor cell inducing immunosuppressive compound is cyclosporin A.

## Patentansprüche (Patentansprüche für folgende(n) Vertragsstaat(en): AT, BE, CH, DE, DK, FR, GB, IT, LI, LU, NL, SE)

1. Zusammensetzung, die enthält
(a) eine Verbindung, die nicht-spezifische (NS) Suppressorzellen induziert, und
(b) eine immunsuppressive Verbindung, die nicht-NS Suppressorzellen induziert.

2. Zusammensetzung nach Anspruch 1, worin die Menge der die nicht-spezifischen Suppressorzellen induzierenden Verbindung ausgewählt ist aus 0,01 - 1000 mg/kg und die Menge der die nicht-NS Suppressorzellen induzierenden immunsuppressiven Verbindung zwischen 0,01 - 20 mg/kg gewählt wird.

3. Zusammensetzung nach Anspruch 1, worin die nicht-spezifsche Suppressorzellen induzierende Verbindung N,N-Diethyl-8,8-dipropyl-2-azaspiro[4,5]-decan-2-propanamin oder N,N-Dimethyl-8,8-dipropyl-2-azaspiro[4,5]-decan-2-propanamin ist und die nicht-NS Suppressorzellen induzierende iminunsuppressive Verbindung folgende ist: FK-506, Spergualin, Rapamycin, RS-61443 oder Cyclosporin A.

4. Zusammensetzung nach Anspruch 1, worin die nicht-spezifische Suppressorzellen induzierende Verbindung N,N-Diethyl-8,8-dipropyl-2-azaspiro[4,5]-decan-2-propanamin ist und die nicht-NS Suppressorzellen induzierende immunsuppressive Verbindung Cyclosporin A ist.

5. Pharmazeutische Zusammensetzung, die eine Zusammensetzung nach einem der Ansprüche 1 bis 4 und einen pharmazeutisch annehmbaren Träger enthält.

6. Zusammensetzung nach einem der Ansprüche 1 bis 5 zur Verwendung als therapeutisch wirksame Substanz.

7. Zusammensetzung nach einem der Ansprche 1 bis 5 zur Verwendung bei der Induzierung einer immunsuppressiven Wirkung.

8. Verwendung einer Zusammensetzung nach einem der Ansprüche 1 bis 5 zur Herstellung eines Arzneimittels zur Verwendung bei der Induzierung einer immunsuppressiven Wirkung.

9. Verwendung einer Verbindung, die nicht-spezifische (NS) Suppressorzellen induziert, zur Herstellung eines Arzneimittels zur Induzierung einer Immunsuppression durch die aufeinanderfolgende oder gleichzeitige Verabreichung einer nicht-NS Suppressorzellen induzierenden immunsuppressiven Verbindung.

10. Verwendung einer immunsuppressiven Verbindung, die nicht-NS Suppressorzellen induziert, zur Herstellung eines Arzneimittels zur Induzierung einer Immunsuppression durch die aufeinanderfolgende oder gleichzeitige Verabreichung einer nicht-spezifische (NS) Suppressorzellen induzierenden Verbindung.

11. Verwendung nach Anspruch 1 oder 2, worin die Menge der nicht-spezifische Suppressorzellen induzierenden Verbindung ausgewählt ist aus 0,01 - 1000 mg/kg und die Menge der nicht-NS Suppressorzellen induzierenden innumsuppressiven Verbindung zwischen 0,01 - 20 mg/kg gewählt wird.

12. Verwendung nach Anspruch 1 oder 2, worin die nicht-spezifische Suppressorzellen induzierende Verbindung N,N-Diethyl-8,8-dipropyl-2-azaspiro[4,5]-decan-2-propanamin oder N,N-Dimethyl-8,8-dipropyl-2-azaspiro[4,5]-decan-2-propanamin ist und die nicht-NS Suppressorzellen induzierende immunsuppressive Verbindung folgende ist: FK-506, Spergualin, Rapamycin, RS-61443 oder Cyclosporin A.

13. Verwendung nach Anspruch 4, worin die nicht-spezifische Suppressorzellen induzierende Verbindung N,N-Diethyl-8,8-dipropyl-2-azaspiro[4,5]-decan-2-propanamin ist und die nicht-NS Suppressorzellen induzierende immunsuppressive Verbindung Cyclosporin A ist.

## Patentansprüche (Patentansprüche für folgende(n) Vertragsstaat(en): ES, GR)

1. Verwendung einer Verbindung, die nicht-spezifische (NS) Suppressorzellen induziert, zur Herstellung eines Arzneimittels zur Induzierung einer Immunsuppression durch die aufeinanderfolgende oder gleichzeitige Verabreichung einer nicht-NS Suppressorzellen induzierenden immunsuppressiven Verbindung.

2. Verwendung einer irnmunsuppressiven Verbindung, die nicht-NS Suppressorzellen induziert, zur Herstellung eines Arzneimittels zur Induzierung einer Immunsuppression durch die aufeinanderfolgende oder gleichzeitige Verabreichung einer nicht-spezifische (NS) Suppressorzellen induzierenden Verbindung.

3. Verwendung nach Anspruch 1 oder 2, worin die Menge der nicht-spezifische Suppressorzellen induzierenden Verbindung ausgewählt ist aus 0,01 - 1000 mg/kg und die Menge der nicht-NS Suppressorzellen induzierenden immunsuppressiven Verbindung zwischen 0,01 - 20 mg/kg gewählt wird.

4. Verwendung nach Anspruch 1 oder 2, worin die nicht-spezifische Suppressorzellen induzierende Verbindung N,N-Diethyl-8,8-dipropyl-2-azaspiro[4,5]-decan-2-propanamin oder N,N-Dimethyl-8,8-dipropyl-2-azaspiro[4,5]-decan-2-propanamin ist und die nicht-NS Suppressorzellen induzierende immunsuppressive Verbindung folgende ist: FK-506, Spergualin, Rapamycin, RS-61443 oder Cyclosporin A.

5. Verwendung nach Anspruch 4, worin die nicht-spezifische Suppressorzellen induzierende Verbindung N,N-Diethyl-8,8-dipropyl-2-azaspiro[4,5]-decan-2-propanamin ist und die nicht-NS Suppressorzellen induzierende immunsuppressive Verbindung Cyclosporin A ist.

## Revendications (Revendications pour l'(les) Etat(s) contractant(s) suivant(s): AT, BE, CH, DE, DK, FR, GB, IT, LI, LU, NL, SE)

1. Composition comprenant:
(a) un composé inducteur de cellules suppressives non spécifiques (NS), et
(b) un composé immunosuppresseur non inducteur de cellules suppressives NS.

2. Compositionn suivant la revendication 1, dans laquelle la quantité de composé inducteur de cellules suppressives non spécifiques est choisie dans la plage de 0,01 à 1000 mg/kg et la quantité de composé immunosuppresseur non inducteur de cellules suppressives NS est choisie dans la plage de 0,01 à 20 mg/kg.

3. Composition suivant la revendication 1, dans laquelle le composé inducteur de cellules suppressives non spécifiques est la N,N-diéthyl-8,8-dipropyl-2-azaspiro[4,5]-décane-2-propanamine ou la N,N-diméthyl-8,8-dipropyl-2-azaspiro[4,5]-décane-2-propanamine et le composé immunosuppresseur non inducteur de cellules suppressives NS est le produit FK-506, la spergualine, la rapamycine, le produit RS-61443 ou la cyclosporine A.

4. Composition suivant la revendicationn 1, dans laquelle le composé inducteur de cellules suppressives non spécifiques est la N,N-diéthyl-8,8-dipropyl-2-azaspiro[4,5]-décane-2-propanamine et le composé immunosuppresseur non inducteur de cellules suppressives NS est la cyclosporine A.

5. Composition pharmaceutique, comprenant une composition suivant l'une quelconque des revendications 1 à 4 et un support acceptable du point de vue pharmaceutique.

6. Composition suivant l'une quelconque des revendications 1 à 5, destinée à être utilisée comme substance thérapeutique active.

7. Composition suivant l'une quelconque des revendications 1 à 5, destinée à être utilisée pour induire une activité immunosuppressive.

8. Utilisation d'une composition suivant l'une quelconque des revendications 1 à 5 dans la préparation d'un médicament destiné à être utilisé pour induire une activité immunosuppressive.

9. Utilisation d'un composé inducteur de cellules suppressives non spécifiques (NS) dans la préparation d'un médicament destiné à induire une immunosuppression avec l'administation consécutive ou simultanée d'un composé immunosuppresseur non inducteur de cellules suppressives NS.

10. Utilisation d'un composé immunosuppresseur non inducteur de cellules suppressives NS dans la préparation d'un médicament destiné à induire une immunosuppression avec l'administration consécutive ou simultanée d'un composé induisant des cellules suppressives non spécifiques (NS).

11. Utilisation suivant la revendication 1 ou 2, dans laquelle la quantité de composé inducteur de cellules suppressives non spécifiques est choisie dans la plage de 0,1 à 1000 mg/kg et la quantité de composé immunosuppresseur non inducteur de cellules suppressives NS est choisie dans la plage de 0,01 à 20 mg/kg.

12. Utilisation suivant la revendication 1 ou 2, dans laquelle le composé inducteur de cellules suppressives non spécifiques est la N,N-diéthyl-8,8-dipropyl-2-azaspiro[4,5]-décane-2-propanamine ou la N,N-diméthyl-8,8-dipropyl-2-azaspiro[4,5]-décane-2-propanamine et le composé immunosuppresseur non inducteur de cellules suppressives NS est le produit FK-506, la spergualine, la rapamycine, le produit RS-61443 ou la cyclosporine A.

13. Utilisation suivant la revendication 4, dans laquelle le composé inducteur de cellules suppressives non spécifiques est la N,N-diéthyl-8,8-dipropyl-2-azaspiro[4,5]-décane-2-propananime et le composé immunosuppresseur non inducteur de cellules suppressives NS est la cyclosporine A.

## Revendications (Revendications pour l'(les) Etat(s) contractant(s) suivant(s): ES, GR)

1. Utilisation d'un composé inducteur de cellules suppressives non spécifiques (NS) dans la préparation d'un médicament destiné à induire une immunosuppression avec l'administation consécutive ou simultanée d'un composé immunosuppresseur non inducteur de cellules suppressives NS.

2. Utilisation d'un composé immunosuppresseur non inducteur de cellules suppressives NS dans la préparation d'un médicament destiné à induire une immunosuppression avec l'administration consécutive ou simultanée d'un composé induisant des cellules suppressives non spécifiques (NS).

3. Utilisation suivant la revendication 1 ou 2, dans laquelle la quantité de composé inducteur de cellules suppressives non spécifiques est choisie dans la plage de 0,1 à 1000 mg/kg et la quantité de composé immunosuppresseur non inducteur de cellules suppressives NS est choisie dans la plage de 0,01 à 20 mg/kg.

4. Utilisation suivant la revendication 1 ou 2, dans laquelle le composé inducteur de cellules suppressives non spécifiques est la N,N-diéthyl-8,8-dipropyl-2-azaspiro[4,5]-décane-2-propanamine ou la N,N-diméthyl-8,8-dipropyl-2-azaspiro[4,5]-décane-2-propanamine et le composé immunosuppresseur non inducteur de cellules suppressives NS est le produit FK-506, la spergualine, la rapamycine, le produit RS-61443 ou la cyclosporine A.

5. Utilisation suivant la revendication 4, dans laquelle le composé inducteur de cellules suppressives non spécifiques est la N,N-diéthyl-8,8-dipropyl-2-azaspiro[4,5]-décane-2-propanamine et le composé immunosuppresseur non inducteur de cellules suppressives NS est la cyclosporine A.
